Europäisches Patentamt

(19) European Patent Office

Office européen des brevets

(11) Veröffentlichungsnummer : **0 168 741**
**B1**

(12) **EUROPÄISCHE PATENTSCHRIFT**

(45) Veröffentlichungstag der Patentschrift :
02.11.89

(21) Anmeldenummer : 85108428.5

(22) Anmeldetag : 08.07.85

(51) Int. Cl.⁴ : **A 61 K 7/06, A 61 K 7/13,**
**A 46 B 11/00, C 08 J 9/00,**
**C 08 J 9/40, A 45 D 24/22**

(54) Werkzeug zur Haarbehandlung.

(30) Priorität : 16.07.84 DE 3426122

(43) Veröffentlichungstag der Anmeldung :
22.01.86 Patentblatt 86/04

(45) Bekanntmachung des Hinweises auf die Patenterteilung : 02.11.89 Patentblatt 89/44

(84) Benannte Vertragsstaaten :
AT BE CH DE FR GB IT LI NL

(56) Entgegenhaltungen :
EP--A-- 0 069 952
EP--A-- 0 157 032
CH--A-- 555 385
DE--A-- 2 737 745
GB--A-- 632 544
GB--A-- 1 596 071
GB--A-- 2 022 415
US--A-- 2 577 921

(73) Patentinhaber : Henkel Kommanditgesellschaft auf
Aktien
Postfach 1100 Henkelstrasse 67
D-4000 Düsseldorf-Holthausen (DE)

(72) Erfinder : Busch, Peter, Dr.
Gottfried-August-Bürger-Strasse
D-4006 Erkrath-Unterbach (DE)
Erfinder : Thiele, Klaus
Rügenweg 5
D-4018 Langenfeld (DE)
Erfinder : Schumann, Henning, Dr.
Sandbrink 18
D-3000 Hannover 51 (DE)
Erfinder : Glasl, Johann, Dr.
Baverter Strasse 58
D-5650 Solingen (DE)

EP 0 168 741 B1

## Beschreibung

Die Erfindung betrifft ein Werkzeug zur kosmetischen Behandlung des menschlichen Haupthaars.

Das Kopfhaar bedarf der ständigen Pflege unter Verwendung mechanischer Werkzeuge wie Kämme, Bürsten oder Striegel zur Entwirrung und Formgebung. Zur Pflege und zur Verbesserung der Frisierbarkeit, des Glanzes und der Fülle werden kosmetische Hilfsmittel wie Haarwässer, Pomaden, Haarkurpräparate, Festiger und Haarspülmittel aufgebracht. Zur Veränderung der Form und Farbe des Haares kann es erwünscht sein, Färbemittel, Bleichmittel oder Wellmittel aufzubringen.

Es hat nicht an Versuchen gefehlt, Werkzeuge zur mechanischen Behandlung und Formgebung des Haares so auszustatten, daß gleichzeitig eine pflegende Wirkung oder eine dekorative Veränderung des Haares erreicht wird. In GB-PS 63 25 44 wird z. B. ein Kamm oder eine Bürste vorgeschlagen, deren Zinken oder Borsten aus einem wasserlöslichen Polymeren, z. B. aus Methylcellulose bestehen, in welchen Farbstoffe oder Bleichmittel fein verteilt oder gelöst sind. Die vorgeschlagene Lösung weist aber erhebliche Nachteile auf, da dieses Gerät nach kurzer Zeit seine Wirkstoffe mit dem Polymeren abgibt und dann für eine weitere Verwendung unbrauchbar ist. Eine Pflegebürste, die ein Reservoir im Bürstenkörper aufweist, ist aus EP-A-97322 bekannt, ein Farbauftragskamm, der das Beladen mit größeren Mengen an Farbmasse erlaubt, ist aus DE-OS 32 21 410 bekannt. Die genannten Geräte sind aber recht kompliziert aufgebaut und eignen sich jeweils nur für spezielle Pflege- oder Behandlungsmittel.

Es bestand daher die Aufgabe, ein universell anwendbares Werkzeug zur mechanischen und zugleich pflegenden oder dekorativen kosmetischen Behandlung des menschlichen Haupthaares zu finden, welches mehrfach wiederverwendbar, einfach herstellbar und zur Aufbringung verschiedener Pflege- und Behandlungsmittel auf das Haar brauchbar ist.

Die gestellte Aufgabe wurde erfindungsgemäß gelöst durch ein Werkzeug zur kosmetischen Haarbehandlung in Form eines Kamms, einer Bürste oder eines Striegels, das dadurch gekennzeichnet ist, daß die mit dem Haar in Kontakt kommenden Zinken, Borsten oder Rippen ganz oder teilweise aus mikroporösen Polymeren bestehen.

Mikroporöse Polymere, die sich für die Herstellung der erfindungsgemäßen Werkzeuge eignen, sind bekannt. Ihre Herstellung wird z. B. in DE-OS 27 37 745 ausführlich beschrieben. Das Prinzip iher Herstellung besteht darin, daß man ein Gemisch aus einem synthetischen, thermoplastischen Polymeren und einer verträglichen Flüssigkeit zur Bildung einer homogenen Lösung auf eine ausreichende Temperatur und eine genügende Zeit lang erhitzt, der Lösung eine gewünschte Form anzunehmen gestattet und die Lösung in der gewünschten Form auf eine Temperatur mit einer ausreichenden Geschwindigkeit abkühlt, so daß unter den thermodynamischen Nicht-Gleichgewichtsbedingungen eine flüssig-flüssig Phasentrennung initiiert wird, das Kühlen dann bis zur Bildung eines festen Körpers fortsetzt und man die Flüssigkeit ganz oder zum überwiegenden Teil aus dem Feststoff zur Bildung der mikroporösen Polymerstruktur entfernt. In DE-OS 27 37 745 sind zahlreiche Beispiele für geeignete Polymere angegeben, die sich auf diese Weise in eine mikroporöse Struktur überführen lassen.

Für die Herstellung der erfindungsgemäßen Werkzeuge sind besonders solche Polymere geeignet, die nach Überführung in die mikroporöse Struktur eine ausreichende mechanische Festigkeit und, bevorzugt auch eine gewisse Biegsamkeit aufweisen, um die Herstellung relativ feiner Zinken, Borsten oder Rippen zu gestatten, die trotzdem noch eine ausreichende Bruchsicherheit aufweisen. Außerdem soll das Polymere eine ausreichende chemische Stabilität gegenüber verschiedenen haarkosmetischen Wirkstoffen aufweisen, d. h. es soll von diesen nicht aufgelöst werden.

Ein besonders gut geeignetes Material ist z. B. ein mikroporöses Polypropylen. Die genannten mikroporösen Polymeren weisen eine relativ homogene, dreidimensionale zellförmige Polymerstruktur auf, d. h. sie bestehen aus einer Vielzahl von im wesentlichen sphärischen Zellen eines mittleren Durchmessers von 0,5 bis etwa 100 Mikron, wobei angrenzende Zellen untereinander durch Poren verbunden sind, die einen kleineren Durchmesser aufweisen als die Zellen. Aufgrund dieser Struktur können diese mikroporösen Polymeren relativ große Mengen flüssiger Substrate aufsaugen und diese langsam und kontrolliert an die mit ihnen in Berührung kommenden Oberflächen, im Falle der erfindungsgemäßen Werkzeuge an die Oberfläche der Haare wieder abgeben.

Obwohl die vorstehend beschriebenen mikroporösen Polymeren zur Herstellung der erfindungsgemäßen Werkzeuge zur kosmetischen Haarbehandlung am besten geeignet sind, sind auch andere, offenporig-mikroporöse Polymeren, z. B. solche die gemäß US-PS 36 15 025 nach dem Phaseninversionsverfahren hergestellt werden, für die Herstellung der erfindungsgemäßen Werkzeuge brauchbar.

Die Herstellung der erfindungsgemäßen Werkzeuge kann z. B. in der Weise erfolgen, daß das mikroporöse Polymere gleich in der Form des Kammes oder eines mit Borsten bzw. Rippen versehenen Bürstenkörpers oder Striegelkörpers hergestellt wird. In diesem Falle wäre es gegebenenfalls angebracht, den Griff oder die Griffpartie mit einer Schutzschicht zu überziehen oder einzukleiden, so daß die Hand nicht direkt mit dem gegebenenfalls mit einem Färbemittel beladenen Polymeren in Berührung kommt.

In einer weiteren Ausführungsform des Erfindungsgegenstandes können z. B. nur die Zinken des Kammes, die Borsten der Bürste oder die Rippen eines striegelähnlichen Werkzeuges aus dem mikroporösen Polymeren ausgebildet sein.

Schließlich kann man ein Werkzeug herkömmlicher Art, also z. B. einen Kamm oder eine Bürste aus beliebigem Material ausreichender Festigkeit mit dem mikroporösen Polymeren wenigstens im Bereich der Zinken oder Borsten überziehen. Dies kann z. B. in der Weise erfolgen, daß man die zu überziehenden Partien des Werkzeugs in die bei der Herstellung der mikroporösen Struktur anfallende Lösung des Polymeren eintaucht, das so beschichtete Material bis zur Ausbildung des festen mikroporösen Körpers abkühlt und das Lösungsmittel aus den Mikroporen entfernt.

Die Mikroporen der erfindungsgemäßen Werkzeuge lassen sich mit wäßrigen oder organischen Lösungen haarkosmetischer Wirkstoffe oder mit flüssigen haarkosmetischen Wirkstoffen dadurch füllen, daß sie mit solchen Lösungen oder Flüssigkeiten über längere Zeit in Berührung gebracht werden. Geeignete Lösungen haarkosmetischer Wirkstoffe sind z. B. wäßrige oder wäßrig-alkoholische Lösungen von antistatisch und avivierend wirksamen quartären Ammoniumsalzen.

Durch Tränken eines erfindungsgemäßen Kammes mit einer Schmelze von Distearyl-dimethylammoniumchlorid oder einer wäßrigen Lösung von Cetyltrimethylammoniumchlorid wurde z. B. ein « Avivagekamm » für zahlreiche Haarbehandlungen erhalten.

Um zu verhindern, daß durch vorzeitige Verdunstung des Lösungsmittels (Wasser, niedere Alkohole) eine gleichmäßige Abgabe des Wirkstoffes an das Haar verhindert wird, kann man die mit Wirkstofflösung getränkten Partien des Werkzeugs feucht halten. Dies ist z. B. durch Abdecken der mikroporösen Partien mit einer undurchlässigen Folie oder dadurch zu erreichen, daß die mikroporösen, mit Wirkstofflösung beladenen Partien des Werkzeugs in einer Lösung des Wirkstoffs eingetaucht, aufbewahrt werden.

Es ist aber meist ausreichend, das an der Luft « ausgetrocknete » oder mit einer Schmelze eines bei Raumtemperatur nicht fließfähigen Behandlungsmittels getränkte Werkzeug durch Eintauchen in Wasser oder ein für den Wirkstoff geeignetes Lösungsmittel vor Gebrauch zu reaktivieren.

Andere, geeignete haarkosmetische Wirkstoffe zur Herstellung der erfindungsgemäßen, mit Wirkstoffen beladenen Werkzeuge sind z. B. kosmetische Öle, Lösungen bzw. Solubilisate von Duftstoffen, sebostatischen Wirkstoffen, Antischuppenmitteln, Vitaminen, Pflanzenextrakten, Kopfhautpflegemitteln, Haarwuchsmitteln, Festigern, Strukturverbesserern (gelösten Polymeren, Glucose usw.).

Eine besonders interessante Möglichkeit bietet sich durch Beladen der mikroporösen Polymeren mit Lösungen direktziehender Haarfarbstoffe. Es können auf diese Weise einzelne Strähnen oder ergraute Haarpartien separat eingefärbt werden. Eine mit einer Farbstofflösung beladene erfindungsgemäße Haarbürste eignet sich für vielfache Anwendungen.

Eine weitere Möglichkeit besteht darin, die Zinken, Borsten oder Rippen der erfindungsgemäßen Werkzeuge mit mit haarverformenden Lösungen zu beladen. Auf diese Weise können einzelne Haarpartien gezielt gekraust oder geglättet werden.

Die nachfolgende Skizze soll den Gegenstand der Erfindung anhand von Beispielen erläutern, ohne ihn hierauf zu beschränken :

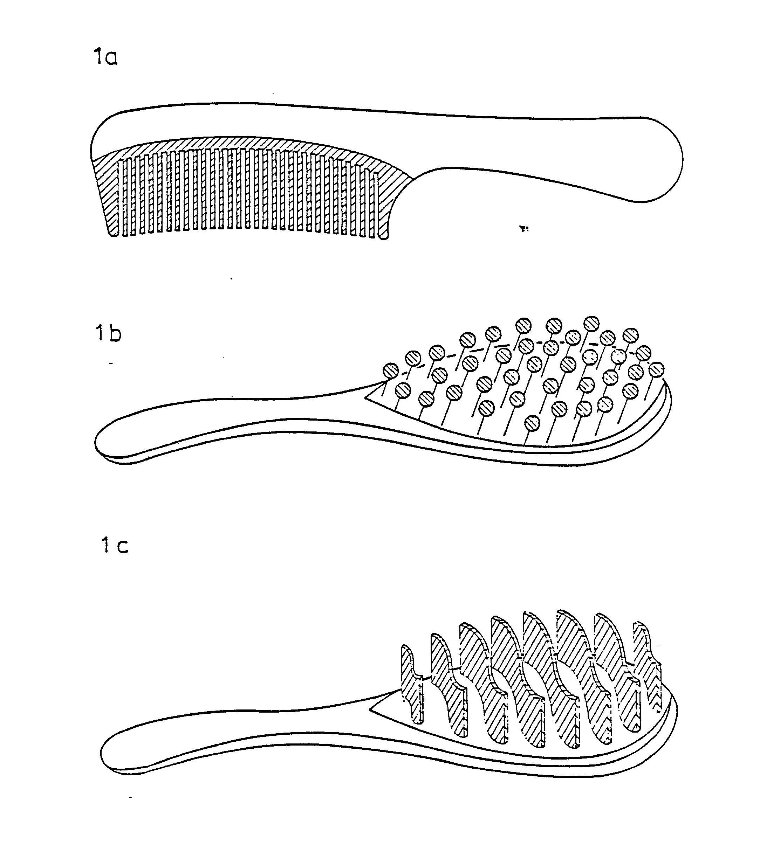

Abbildung 1a : Kamm, die schraffierten Teile sind aus mikroporösem Polypropylen ausgeführt.

Abbildung 1b : Bürste, die Borsten sind aus mikroporösem Polypropylen ausgeführt.

Abbildung 1c : Striegelähnliches Werkzeug ; die Rippen sind aus mikroporösem Polypropylen ausgeführt.

**Patentansprüche** (für die Vertragsstaaten : BE, NL)

1. Werkzeug zur kosmetischen Haarbehandlung in Form eines Kamms, einer Bürste oder eines Striegels, dadurch gekennzeichnet, daß die mit dem Haar in Kontakt kommenden Zinken, Borsten oder Rippen ganz oder teilweise aus mikroporösen Polymeren bestehen.

2. Werkzeug nach Anspruch 1, dadurch gekennzeichnet, daß die Mikroporen mit Lösungen haarkosmetischer Behandlungsmittel oder flüssigen haarkosmetischen Wirkstoffen gefüllt sind.

3. Werkzeug nach Anspruch 1 oder 2, dadurch gekennzeichnet, daß die Mikroporen mit der Lösung einer zur Haaravivage geeigneten Schmelze oder Lösung einer quartären Ammoniumverbindung gefüllt sind.

4. Werkzeug nach Anspruch 1 oder 2, dadurch gekennzeichnet, daß die Mikroporen mit der Lösung eines direktziehenden Haarfarbstoffes gefüllt sind.

**Patentansprüche** (für die Vertragsstaaten : AT, CH, DE, FR, GB, IT, LI)

1. Werkzeug zur kosmetischen Haarbehandlung in Form eines Kamms, einer Bürste oder eines Striegels, dadurch gekennzeichnet, daß die mit dem Haar in Kontakt kommenden Zinken, Borsten oder Rippen ganz oder teilweise aus mikroporösen Polymeren bestehen, welches jedoch keinen, mit porösem Material gefüllten Hohlraum aufweist, der mit den Zinken, Borsten oder Rippen in Verbindung steht.

2. Werkzeug nach Anspruch 1, dadurch gekennzeichnet, daß die Mikroporen mit Lösungen haarkosmetischer Behandlungsmittel oder flüssigen haarkosmetischen Wirkstoffen gefüllt sind.

3. Werkzeug nach Anspruch 1 oder 2, dadurch gekennzeichnet, daß die Mikroporen mit der Lösung einer zur Haaravivage geeigneten Schmelze oder Lösung einer quartären Ammoniumverbindung gefüllt sind.

4. Werkzeug nach Anspruch 1 oder 2, dadurch gekennzeichnet, daß die Mikroporen mit der Lösung eines direktziehenden Haarfarbstoffes gefüllt sind.

**Claims** (for the Contracting States : AT, CH, DE, FR, GB, IT, LI)

1. An implement for the cosmetic treatment of hair in the form of a comb, a brush or a currycomb, characterized in that the teeth, bristles or ribs coming into contact with the hair consist completely or partly of microporous polymers, but with no hollow space filled with porous material which communicates with the teeth, bristles or ribs.

2. An implement as claimed in claim 1, characterized in that the micropores are filled with solutions of hair-cosmetic treatment preparations or liquid hair-cosmetic agents.

3. An implement as claimed in claim 1 or 2, characterized in that the micropores are filled with a solution of a melt or solution of a quaternary ammonium compound suitable for conditioning hair.

4. An implement as claimed in claim 1 or 2, characterized in that the micropores are filled with a solution of a substantive dye.

**Claims** (for the Contracting States : BE and NL)

1. An implement for the cosmetic treatment of hair in the form of a comb, a brush or a currycomb, characterized in that the teeth, bristles or ribs coming into contact with the hair consist completely or partly of microporous polymers.

2. An implement as claimed in claim 1, characterized in that the micropores are filled with solutions of hair-cosmetic treatment preparations or liquid hair-cosmetic agents.

3. An implement as claimed in claim 1 or 2, characterized in that the micropores are filled with a solution of a melt or solution of a quaternary ammonium compound suitable for conditioning hair.

4. An implement as claimed in claim 1 or 2, characterized in that the micropores are filled with a solution of a substantive dye.

**Revendications** (pour les Etats contractants : AT, CH, DE, FR, GB, IT, LI)

1. Instrument pour le traitement cosmétique des cheveux sous la forme d'un peigne, d'une brosse ou d'une brosse à lamelles, caractérisé en ce que les dents, poils ou lamelles entrant en contact avec la chevelure sont constitués totalement ou partiellement de polymères microporeux, qui ne présente cependant pas de creux rempli de matière poreuse en liaison avec les dents, les poils ou les lamelles.

2. Instrument selon la revendication 1, caractérisé en ce que les micropores sont remplis de solutions de produits de traitement cosmétique des cheveux ou de substances actives cosmétiques capillaires liquides.

3. Instrument selon la revendication 1 ou 2, caractérisé en ce que les micropores sont remplis d'une solution d'une masse fondue ou d'une solution d'un composé ammonium quaternaire convenant pour l'avivage de la chevelure.

4. Instrument selon la revendication 1 ou 2, caractérisé en ce que les micropores sont remplis d'une solution d'une teinture capillaire montant directement sur la fibre.

**Revendications** (pour les Etats contractants : BE et NL)

1. Instrument pour le traitement cosmétique des cheveux sous la forme d'un peigne, d'une brosse ou d'une brosse à lamelles, caractérisé en ce que les dents, poils ou lamelles entrant en contact avec la chevelure sont constitués totalement ou partiellement de polymères microporeux.

2. Instrument selon la revendication 1, caractérisé en ce que les micropores sont remplis de solutions de produits de traitement cosmétique des cheveux ou de substances actives cosmétiques capillaires liquides.

3. Instrument selon la revendication 1 ou 2, caractérisé en ce que les micropores sont remplis d'une solution d'une masse fondue ou d'une solution d'un composé ammonium quaternaire convenant pour l'avivage de la chevelure.

4. Instrument selon la revendication 1 ou 2, caractérisé en ce que les micropores sont remplis d'une solution d'une teinture capillaire montant directement sur la fibre.

1a

1b

1c